# DEMANDE DE BREVET EUROPEEN

(11) **EP 1 902 752 A1**
(43) Date de publication de la demande: **26.03.2008**
(21) Numéro de dépôt: 06358016.1
(22) Date de dépôt: 19.09.2006
(51) Int. Cl.: A61Q 9/04, A61K 8/19, A61K 8/25, A61K 8/26, A61K 8/46

(54) **Procédé cosmetique pour éliminer les poils du corps utilisant une composition épilatoire se presentant sous forme de poudre**

(71) Demandeur: Chorfi, Radia, 13003 Marseille (FR)
(72) Inventeur: Chorfi, Radia, 13003 Marseille (FR)
(74) Mandataire: Roman, Michel

(57) **Abrégé**

La présente invention a pour objet un procédé cosmétique pour éliminer temporairement les poils du corps, utilisant une composition dépilatoire se présentant sous forme de poudre.
Selon l'invention, la composition dépilatoire comprend du thioglycolate de calcium, de l'hydroxyde de calcium et une charge granuleuse acceptable en cosmétique lui conférant un aspect de poudre destinée à être mélangée avec un solvant acceptable en cosmétique pour former une crème ayant un pH de 9 à 12,5.
Cette invention permet de proposer une composition dépilatoire dont le procédé de fabrication est simple à mettre en oeuvre et dont l'utilisation est inhabituelle par rapport aux produits similaires existants et peut donc être plaisante pour l'utilisateur.

## Description

La présente invention a pour objet une composition dépilatoire se présentant sous forme de poudre ainsi que son utilisation dans un traitement en cosmétique pour éliminer temporairement les poils du corps.

Les poils sont constitués principalement de protéines fibreuses dénommées kératine reliées entre elles par des ponts disulfures.
Il est commun d'employer dans des crèmes dépilatoires de l'acide thioglycolique ou l'un de ses sels comme agent actif capable de rompre ces ponts disulfures et dissoudre les poils. Habituellement, ce composé est mélangé avec un hydroxyde de métal alcalino-terreux permettant d'ajuster le pH de la composition afin que l'agent actif dépilatoire pénètre rapidement dans la structure fibreuse des poils.
Généralement, le pH de telles crèmes est de 9 à 12.5. En dessous d'un pH 9, l'action dépilatoire est trop lente et au-dessus d'un pH 12.5, la crème risque de provoquer des dommages sur la peau.

En principe, la crème dépilatoire est appliquée à froid sur la zone du corps à traiter, par exemple sur les jambes ou au niveau des aisselles. Après avoir laissé la crème en contact avec la peau entre 5 et 20 minutes, les poils se dissolvent et l'utilisateur n'a plus qu'à rincer la zone dépilée à l'eau claire.

Les crèmes dépilatoires que l'on trouve actuellement dans le commerce ne sont pourtant pas utilisées de manière totalement satisfaisante.
Un premier inconvénient réside dans le fait que la fluidité de la crème est imposée à l'utilisateur. Selon les préférences de ce dernier, il peut la trouver soit trop liquide soit trop pâteuse pour être utilisée convenablement et agréablement.
Un autre inconvénient est lié au conditionnement en tube ou en pot des crèmes dépilatoires existantes. À chaque utilisation, de l'air rentre dans le tube ou le pot, ce qui favorise l'introduction de microbes ou de bactéries dégradant, à terme, les propriétés cosmétiques de la crème.
Le procédé industriel de fabrication, depuis le mélange des différents composants jusqu'au conditionnement de la crème dépilatoire dans un tube, est également complexe et nécessite des installations coûteuses.

La présente invention a pour but de pallier cet état des choses notamment du fait qu'elle permet de proposer une composition dépilatoire dont les propriétés et les caractéristiques cosmétiques sont conservées dans des conditions optimales avec le temps.
Un autre but de l'invention est de proposer une composition dont la fluidité est déterminée par l'utilisateur, selon sa convenance, avant le traitement dépilatoire.
L'invention a encore pour but de proposer une composition dépilatoire dont le prix de revient à la vente est inférieur à celui des crèmes dépilatoires que l'on trouve habituellement dans le commerce.
L'invention a également pour but de proposer une composition dépilatoire dont le procédé de fabrication est simple à mettre en oeuvre et dont l'utilisation est inhabituelle par rapport aux produits similaires existants et peut donc être plaisante pour l'utilisateur.

A la connaissance de la demanderesse, il n'existe pas à ce jour de composition dépilatoire qui se présente sous forme de poudre et qu'un utilisateur peut mélanger avec de l'eau douce pour former une crème dépilatoire à sa convenance.

Les buts de l'invention sont ainsi atteints par une composition dépilatoire destinée à être mélangée avec un solvant acceptable en cosmétique pour former une crème ayant un pH de 9 à 12,5, ladite composition comprenant un sel d'acide thioglycolique, un hydroxyde de métal alcalino-terreux, une charge granuleuse acceptable en cosmétique lui conférant un aspect de poudre. Étant sèche et granuleuse, cette composition se conserve mieux que les crèmes dépilatoires fluides connues. En outre, la composition dépilatoire selon l'invention peut être facilement stockée et conditionnée, notamment sous forme de sachet.

Selon une caractéristique préférée de l'invention, la charge granuleuse est du talc pour apporter à la peau une sensation de douceur agréable au toucher après la dépilation. Dans une variante de réalisation, la charge granuleuse est de l'argile.

Selon une autre caractéristique permettant de dissoudre rapidement et efficacement le poil, le sel d'acide thioglycolique est du thioglycolate de calcium dosé de 2 à 8 % poids/poids et avantageusement l'hydroxyde de métal alcalino-terreux est de l'hydroxyde de calcium dosé de 3 à 9 % poids/poids.

En vue de laisser la peau douce et hydratée après la dépilation et notamment pour que des personnes ayant la peau sensible puisse l'utiliser, la composition objet de l'invention peut en outre comprendre de l'urée, dosé de 2 à 10 % poids/poids et du sorbitol ou du kaolin, dosé de 5 à 15 % poids/poids.

Préférentiellement, la composition est constituée de 4 % poids/poids de thioglycolate de calcium, 6 % poids/poids d'hydroxyde de calcium, 5 % poids/poids d'urée, 10 % poids/poids de sorbitol ou de kaolin, 0 à 1 % poids/poids de parfum, qsp 100 % de talc.

Selon un procédé de traitement cosmétique permettant d'éliminer les poils du corps et laisser repousser les poils après traitement :
- on mélange la composition objet de l'invention avec un solvant acceptable en cosmétique jusqu'à obtenir une crème de fluidité souhaitée ;
- on applique la crème obtenue sur la zone du corps à traiter ;
- on laisse la crème au contact de la peau entre 5 et 10 minutes ;
- on élimine la crème de la surface de la peau en rinçant à l'eau douce.

D'autres caractéristiques et avantages de la présente invention ressortiront mieux à la lecture de la description suivante, faite à titre d'exemple indicatif et non limitatif.

L'agent dépilatoire utilisé est choisi parmi les sels de l'acide thioglycolique comme le thioglycolate de magnésium, le thioglycolate de potassium, le thioglycolate de calcium ou le thioglycolate de sodium. Préférentiellement, on utilise du thioglycolate de calcium pouvant éventuellement être associé à du thioglycolate de sodium.

On peut utiliser comme hydroxyde de métal alcalino-terreux de l'hydroxyde de sodium ou de l'hydroxyde de calcium. On utilise préférentiellement de l'hydroxyde de calcium lorsque l'agent dépilatoire est à base de thioglycolate de calcium. En présence d'hydroxyde de calcium, le pH va être maintenu entre 9 et 12.5 pour favoriser la pénétration du thioglycolate de calcium au niveau des protéines fibreuses du poil.

Pour donner à la composition un aspect de poudre, on utilise une charge granuleuse minérale ou organique. Comme charge minérale on peut utiliser par exemple du talc, de la silice ou de l'argile. Comme charge organique on peut utiliser par exemple des micro-billes éventuellement parfumées.
On préfère l'emploi du talc pour ses propriétés protectrices, modifiant la sensation de toucher après emploi, notamment en apportant une sensation de glissement et de douceur. Le talc utilisé comporte en outre des propriétés apaisantes.

On ajoute préférentiellement à la composition objet de l'invention un humectant, avantageusement de l'urée, pour maintenir la teneur en eau dans la peau. L'urée a une action amollissante sur les couches cornées de la peau et de ce fait la rend souple et améliore la sensation de toucher après la dépilation.

On peut également ajouter un agent hydratant qui est avantageusement du sorbitol ou, de manière équivalente, du kaolin.

Pour rendre l'utilisation de la composition objet de l'invention plus agréable, du parfum ou d'autres fragrances sont avantageusement mélangés aux autres constituants pour masquer notamment l'odeur des sels d'acide thioglycolique utilisés.

D'autres composants comme des tensioactifs ou d'autres éléments habituellement utilisés en cosmétologie pour le soin de la peau peuvent également être utilisés.

Selon une caractéristique essentielle de l'invention, chaque composant se présente sous la forme de poudre soluble totalement ou en partie dans de l'eau douce, chaude ou froide, ou dans tout autre solvant acceptable en cosmétique.

La composition selon l'invention est avantageusement constituée des composants suivants, dosés dans les proportions suivantes :

| Composition 1 | |
|---|---|
| Composants | Variation concentration (% poids/poids) |
| Thioglycolate de calcium | 2 - 8 |
| Hydroxyde de calcium | 3-9 9 |
| Urée | 2-10 |
| Sorbitol | 5-15 |
| Parfum | 0 - 1 |
| Talc | qsp 100 |

Selon un mode préféré de réalisation, la composition est constituée approximativement de :

| Composition 2 | |
|---|---|
| Composants | Concentration (% poids/poids) |
| Thioglycolate de calcium | 4 |
| Hydroxyde de calcium | 6 |
| Urée | 5 |
| Sorbitol | 10 |
| Parfum | 0.5 |
| Talc | qsp 100 |

L'ensemble des composants se trouve aisément dans le commerce et il suffit de les mélanger pour obtenir la composition selon l'invention. Le procédé de fabrication est donc simple et ne nécessite pas de matériel onéreux. Le dosage des différents composés est en outre plus simple que celui des crèmes dépilatoires existantes.
La composition peut en outre être facilement stockée pendant un long moment et transportée sans risque de détérioration.

Une fois que la composition est préparée, elle est avantageusement conditionnée dans des sachets de 100 à 200g, en papier ou en plastique facilement ouvrable ou muni d'une ouverture spécifique de manière à ce que l'utilisateur puisse aisément récupérer la poudre avant utilisation.
La quantité de composition présente dans chaque sachet est déterminée pour qu'avec un ou deux sachets, l'utilisateur puisse dépiler par exemple les deux jambes et/ou les deux aisselles.

Le produit proposé à la vente peut être constitué d'une boîte en carton à faible coût de revient dans laquelle sont présents un nombre déterminé de sachets.
Dans une variante de réalisation, la composition objet de l'invention est vendue en même temps qu'un solvant acceptable en cosmétique, par exemple de l'eau distillée parfumée ou tout autre solvant capable de dissoudre totalement ou en partie les composants de ladite composition pour former une crème.

D'autres modes de conditionnement peuvent être prévus, et la composition peut notamment est conditionnée dans des récipients de plus grandes capacités en plastique ou en carton, dans des sacs, ou tous autres contenants appropriés pour recevoir des poudres.

Pour utiliser la composition objet de l'invention, il suffit de la mélanger avec un solvant acceptable en cosmétique et adapté aux différents composants constitutifs, jusqu'à l'obtention d'une crème. Par exemple, pour les compositions 1 ou 2 décrites précédemment, un mélange avec de l'eau douce, chaude ou froide, convient.
Le terme crème doit être entendu dans la présente invention comme une préparation onctueuse ou mousseuse.
Le mélange s'effectue préférentiellement avec une petite spatule en bois ou en plastique présente dans le conditionnement proposé à la vente.
La fluidité de la crème obtenue est déterminée selon le souhait de l'utilisateur : si la crème est trop fluide, il suffit de rajouter une petite quantité de la composition et si la crème est trop pâteuse, il suffit de rajouter une petite quantité d'eau ou de solvant.
La crème ainsi obtenue est étalée à la main ou avec une spatule sur la zone du corps à dépiler.

Après avoir laissé la crème en contact avec la peau pendant 5 à 10 minutes, on rince soigneusement à l'eau pour éliminer la crème et les poils dissous.

## Revendications

1. Procédé cosmétique pour éliminer les poils du corps et laisser repousser les poils après traitement, **se caractérisant par le fait qu'**il consiste à :
- mélanger du thioglycolate de calcium, de l'hydroxyde de calcium, une charge granuleuse acceptable en cosmétique pour former une composition dépilatoire se présentant sous forme de poudre, chaque composant se trouvant sous la forme de poudre soluble totalement ou en partie dans un solvant acceptable en cosmétologie ;
- mélanger la composition dépilatoire obtenue avec un solvant acceptable en cosmétique jusqu'à l'obtention d'une crème de fluidité souhaitée et ayant un pH de 9 à 12.5 ;
- appliquer la crème obtenue sur la zone du corps à traiter ;
- laisser la crème au contact de la peau entre 5 et 10 minutes ;
- éliminer la crème de la surface de la peau en rinçant à l'eau douce.

2. Procédé selon la revendication 1, dans lequel on utilise comme charge granuleuse du talc.

3. Procédé selon la revendication 1, dans lequel on utilise comme charge granuleuse de l'argile.

4. Procédé selon la revendication 1, dans lequel on utilise comme charge granuleuse de la silice.

5. Procédé selon la revendication 1, dans lequel on utilise comme charge granuleuse des micro-billes parfumées.

6. Procédé selon l'une des revendications précédentes, dans lequel le thioglycolate de calcium est dosé de 2 à 8 % poids/poids.

7. Procédé selon l'une des revendications précédentes, dans lequel l'hydroxyde de calcium est dosé de 3 à 9 % poids/poids.

8. Procédé selon l'une des revendications précédentes, dans lequel la composition dépilatoire comprend en outre :
- de l'urée, dosée de 2 à 10 % poids/poids ;
- du sorbitol ou du kaolin, dosé de 5 à 15 % poids/poids.

9. Procédé selon l'une des revendications précédentes, dans lequel la composition dépilatoire est constituée de :
- 4 % poids/poids de thioglycolate de calcium ;
- 6 % poids/poids d'hydroxyde de calcium ;
- 5 % poids/poids d'urée ;
- 10 % poids/poids de sorbitol ou de kaolin ;
- 0 à 1 % poids/poids de parfum ;
- qsp 100 % de talc.

10. Procédé selon l'une des revendications 1 à 8, dans lequel le thioglycolate de calcium est associé à du thioglycolate de sodium.
